# EUROPEAN PATENT SPECIFICATION

(11) **EP 2 283 804 B1**
(45) Date of publication and mention of the grant of the patent: **01.08.2012**
(21) Application number: 09167690.8
(22) Date of filing: 12.08.2009
(51) Int. Cl.: A61K 8/19, A61K 8/42, A61Q 1/00, A61Q 1/02

(54) **Cosmetic compositions with gold particles and alkyl acid monoethanolamides**
Kosmetische Zusammensetzungen mit Goldpartikeln und Alkylsäure-Monoethanolamiden
Compositions cosmétiques dotées de particules d'or et monoéthanolamides d'acide alkylique

(43) Date of publication of application: 16.02.2011
(73) Proprietor: Unilever PLC, A Company Registered in England and Wales under Company no. 41424, London EC4Y 0DY Greater London (GB); UNILEVER NV, 3013 AL Rotterdam (NL)
(72) Inventor: Ang, Endang Saraswati Angka, Changning District, Shanghai 20335 (CN); Wu, Ji Wei, Changning District, Shanghai 20335 (CN); Zhang, Qian, Changning District, Shanghai 20335 (CN)
(74) Representative: Acham, Nicholas Clive

(56) References cited:
- WO-A1-86/00799
- DE-U1-202007 004 249
- FR-A1- 2 554 715
- ANONYMOUS: "SCHERCOMID AME-75" The Lubrizol Corporation LUBRIZOL TECHNICAL DATA SHEET 28 February 2007 (2007-02-28), XP002576213 Retrieved from the Internet: URL:http://www.lubrizol.co.kr/WorkArea/lin kit.aspx?LinkIdentifier=id&ItemID=32006> [retrieved on 2010-03-16]
- EMMERT R: "Quantification of the soft focus effect" COSMETICS & TOILETRIES, WHEATON, IL, US, vol. 111, no. 7, 1 July 1996 (1996-07-01), pages 57-61, XP009029669 ISSN: 0361-4387

## Description

### BACKGROUND OF THE INVENTION

The invention relates to compositions for improving the appearance of skin, particularly to provide good coverage over imperfections such as pores and uneven skin tone, while retaining a natural skin appearance.

Cover-up of imperfect skin can be effected by applying a matte finish. An opaque cosmetic layer hides any unsightly topography underneath. The matte finish overcomes the shiny effect engendered by greasy skin, particularly under hot and humid conditions. Absorbent fillers such as talc, silica, kaolin and other inorganic particulates have been used to achieve the effect by their optical properties.

Imperfect skin can also be hidden in two other ways through manipulation of light transmission. In the first, components of the color cosmetic may simply reflect light back toward the source. An alternative approach is referred to as achieving a soft focus effect. Here the incoming light is distorted by scattering (lensing). Components of the color cosmetic in this mechanism operate as lenses to bend and twist light into a variety of directions.

While it is desirable to hide imperfect skin through a matte effect, there is also a desire to achieve a healthy skin radiance. A cosmetic covering that is too opaque hides the skin under a paint-like coating. Imperfections are hidden but there is no radiance. Where light transmission is insufficiently hindered, the opposite occurs. Here the glow may be healthy but aesthetically displeasing skin topography and color may now be apparent.

US 5 997 890 (Sine et al.), US 5 972 359 (Sine et al.), and US 6 174 533 B1 (SaNogueira, Jr.) are all directed to topical compositions to provide good coverage of skin imperfections. The solution proposed by these documents is the use of a metal oxide with a refractive index of at least about 2 and a neat primary particle size of from about 100 to about 300 nm. Preferred particulates are titanium dioxide, zirconium oxide and zinc oxide.

The present inventors have investigated the possibility of utilizing precious metal particulates such as gold to achieve soft focus effects. The art has described many cosmetic systems employing precious metals. For instance, US 2 111 912 (Govett) reports a small quantity of colloidal gold suspended by an emulsifying agent in a cream. WO 86/00799 (Falsarella) discloses cosmetic products containing pure or alloy gold in the form of flakes in sizes ranging from 3 to 30 µm. US 4 828 825 (Weber et al.) describes a skin composition wherein fine particles of mica are coated with an infrared reflecting material selected from a group, one of which may be a noble metal. WO 03/020226 (Carrion et al.) reveals hair compositions having nanoparticles of metallic substances that may include silver, gold, platinum, palladium and nickel. German patent application 196 25 560 A1 (Ukleja et al.) reports mixing of gold flakes having thickness 0.1-50 micron into general cosmetic products. US 2003/0019501 A1 (Hirota et al.) discloses glittering cosmetics comprising scaly glittering particles which may be an alloy of silver or gold and having a smooth metal surface.

None of the references reveal any potential for gold particles as useful in providing soft focus effects. Perhaps this oversight arises from the costly nature of precious metals. Economically it is unjustifiable to formulate with sufficient amount of precious metal to achieve the desired effect. Thus there is a challenge to incorporating gold and other precious metal particles at relatively economic concentration levels while still eliciting a soft focus benefit.

### SUMMARY OF THE INVENTION

A cosmetic composition is provided which includes:
i) from 0.00001 to 0.05% by weight of gold partides;
ii) from 0.5 to 4% by weight of an alkanolamide having structure (I) 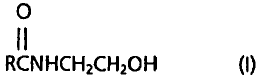 wherein R is a C₁-C₃ radical selected from the group consisting of methyl, ethyl, propyl, and isopropyl; and
iii) a cosmetically acceptable carrier which is water in an amount from 5 to 95% by weight of the composition.

### DETAILED DESCRIPTION OF THE INVENTION

Now it has been found that a soft focus effect can be elicited from even small amounts of gold particles through combination with a benefit enhancing amount of an alkanolamide.

Gold particles according to the present invention can come in different sizes and shapes. The particles may be in spherical, platelet, flake, or irregular shape form. Flake form is most useful. Average particle size may range from 1 to 1000 micron, preferably from 10 to 1,000 micron, optimally from 100 to 800 micron. Amounts of the gold particles may range from 0.00001 to 0.05%, preferably from 0.0005 to 0.05%, more preferably from 0.0003 to 0.03% and optimally from 0.0002 to 0.02% by weight of the composition.

The gold particles may be 24 karat, or gold alloys with other metals such as tin, copper, nickel, titanium, silver or combinations thereof (e.g. 22 karat). Gold particles according to the present invention may also be gold compounds rather than in elemental form. For instance gold chloride, gold sulphate, gold nitrate, gold phosphate, gold borate or organo gold complexes are herein encompassed. Nonetheless most preferred is elemental gold. Particles wherein the gold particles are not supported on carrier particles nor are coatings on carrier particles (e.g. mica of titanium dioxide) are especially preferred for the present invention.

Alkanolamides of the present invention will have a structure (I) of the general formula: 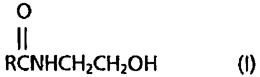 wherein R is a C₁-C₃ radical selected from the group consisting of methyl, ethyl, propyl and isopropyl.

Most preferred is where R is methyl which provides the material known as acetamide monoethanolamine. This material is commercially available from Croda Incorporated. under the trademark Incromectant®.

Compositions of this invention will also include a cosmetically acceptable carrier. Amounts of the carrier may range from 1 to 99.9%, preferably from 70 to 95%, optimally from 80 to 90% by weight of the composition. Among the useful carriers are water, emollients, fatty acids, fatty alcohols, thickeners and combinations thereof. The compositions are aqueous, especially water and oil emulsions of the W/O or O/W or triplex W/O/W variety. Water is present in amounts ranging from 5 to 95%, preferably from 20 to 70%, optimally from 35 to 60% by weight of the composition.

Emollient materials may serve as cosmetically acceptable carriers. These may be in the form of silicone oils, natural or synthetic esters and hydrocarbons. Amounts of the emollients may range anywhere from 0.1 to 95%, preferably between 1 and 50% by weight of the composition.

Silicone oils may be divided into the volatile and non-volatile variety. The term "volatile" as used herein refers to those materials which have a measurable vapor pressure at ambient temperature. Volatile silicone oils are preferably chosen from cyclic (cyclomethicone) or linear polydimethylsiloxanes containing from 3 to 9, preferably from 4 to 5 silicon atoms.

Non-volatile silicone oils useful as an emollient material include polyalkyl siloxanes, polyalkylaryl siloxanes and polyether siloxane copolymers. The essentially non-volatile polyalkyl siloxanes useful herein include, for example, polydimethyl siloxanes with viscosities of from about 5 x 10⁻⁶ to 0.1 m²/s at 25°C. Among the preferred non-volatile emollients useful in the present compositions are the polydimethyl siloxanes having viscosities from about 1 x 10⁻⁵ to about 4 x 10⁻⁴ m²/s at 25°C.

Another class of non-volatile silicones are emulsifying and non-emulsifying silicone elastomers. Representative of this category is dimethicone/vinyl dimethicone crosspolymer available as Dow Corning 9040, General Electric SFE 839 and Shin-Etsu KSG-18. Silicone waxes such as Silwax WS-L (dimethicone copolyol laurate) may also be useful.

Among the ester emollients are:
a) Alkyl esters of saturated fatty acids having 10 to 24 carbon atoms. Examples thereof include behenyl neopentanoate, isononyl isonanonoate, isopropyl myristate and octyl stearate.
b) Ether-esters such as fatty acid esters of ethoxylated saturated fatty alcohols.
c) Polyhydric alcohol esters. Ethylene glycol mono- and di-fatty acid esters, diethylene glycol mono- and di-fatty acid esters, polyethylene glycol (200-6000) mono- and di-fatty acid esters, propylene glycol mono- and di-fatty acid esters, polypropylene glycol 2000 monostearate, ethoxylated propylene glycol monostearate, glyceryl mono- and di-fatty acid esters, polyglycerol poly-fatty esters, ethoxylated glyceryl mono-stearate, 1,3-butylene glycol monostearate, 1,3-butylene glycol distearate, polyoxyethylene polyol fatty acid ester, sorbitan fatty acid esters, and polyoxyethylene sorbitan fatty acid esters are satisfactory polyhydric alcohol esters. Particularly useful are pentaerythritol, trimethylolpropane and neopentyl glycol esters of C₁-C₃₀ alcohols.
d) Wax esters such as beeswax, spermaceti wax and tribehenin wax.
e) Sugar ester of fatty acids such as sucrose polybehenate and sucrose polycottonseedate.

Natural ester emollients principally are based upon mono-, di- and tri- glycerides. Representative glycerides include sunflower seed oil, cottonseed oil, borage oil, borage seed oil, primrose oil, castor and hydrogenated castor oils, rice bran oil, soybean oil, olive oil, safflower oil, shea butter, jojoba oil and combinations thereof. Animal derived emollients are represented by lanolin oil and lanolin derivatives. Amounts of the natural esters may range from 0.1 to 20% by weight of the compositions.

Hydrocarbons which are suitable cosmetically acceptable carriers include petrolatum, mineral oil, C₁₁-C₁₃ isoparaffins, polybutenes and, especially, isohexadecane available commercially as Permethyl 101A from Presperse Incorporated.

Fatty acids having from 10 to 30 carbon atoms may also be suitable as cosmetically acceptable carriers. Illustrative of this category are pelargonic, lauric, myristic, palmitic, stearic, isostearic, oleic, linoleic, linolenic, hydroxystearic and behenic acids.

Fatty alcohols having from 10 to 30 carbon atoms are another useful category of cosmetically acceptable carrier. Illustrative of this category are stearyl alcohol, lauryl alcohol, myristyl alcohol, oleyl alcohol and cetyl alcohol.

Thickeners can be utilized as part of the cosmetically acceptable carrier of compositions according to the present invention. Typical thickeners include crosslinked acrylates (e.g. Carbopol 982®), hydrophobically-modified acrylates (e.g. Carbopol 1382®), polyacrylamides (e.g. Sepigel 305®), acryloylmethylpropane sulfonic acid/salt polymers and copolymers (e.g. Aristoflex HMB® and AVC®), cellulosic derivatives and natural gums. Among useful cellulosic derivatives are sodium carboxymethylcellulose, hydroxypropyl methocellulose, hydroxypropyl cellulose, hydroxyethyl cellulose, ethyl cellulose and hydroxymethyl cellulose. Natural gums suitable for the present invention include guar, xanthan, sclerotium, carrageenan, pectin and combinations of these gums. Inorganics may also be utilized as thickeners, particularly clays such as bentonites and hectorites, fumed silicas, calcium carbonate and silicates such as magnesium aluminum silicate (Veegum®). Amounts of the thickener may range from 0.0001 to 10%, usually from 0.001 to 1%, optimally from 0.01 to 0.5% by weight of the composition.

Humectants may be employed in the present invention. These are generally polyhydric alcohol-type materials. Typical polyhydric alcohols include glycerol, propylene glycol, dipropylene glycol, polypropylene glycol, polyethylene glycol, sorbitol, hydroxypropyl sorbitol, hexylene glycol, 1,3-butylene glycol, isoprene glycol, 1,2,6-hexanetriol, ethoxylated glycerol, propoxylated glycerol and mixtures thereof. The amount of adjunct humectant may range anywhere from 0.5 to 50%, preferably between 1 and 15% by weight of the composition.

Compositions of the present invention may be in any form. These forms may include lotions, creams, roll-on formulations, sticks, mousses, aerosol and non-aerosol sprays and fabric (e.g. non-woven textile) applied formulations.

Surfactants may also be present in compositions of the present invention. Total concentration of the surfactant when present may range from 0.1 to 30%, preferably from 0.1 to 15%, optimally from 0.5 to 2% by weight of the composition. The surfactant may be selected from the group consisting of anionic, nonionic, cationic and amphoteric actives. Particularly preferred nonionic surfactants are those with a C₁₀-C₂₀ fatty alcohol or acid hydrophobe condensed with from 2 to 100 moles of ethylene oxide or propylene oxide per mole of hydrophobe; C₂-C₁₀ alkyl phenols condensed with from 2 to 20 moles of alkylene oxide; mono- and di-fatty acid esters of ethylene glycol; fatty acid monoglyceride; sorbitan, mono- and di- C₈-C₂₀ fatty acids; and polyoxyethylene sorbitan as well as combinations thereof. Alkyl polyglycosides and saccharide fatty amides (e.g. methyl gluconamides) and trialkylamine oxides are also suitable nonionic surfactants.

Preferred anionic surfactants include soap, alkyl ether sulfates and sulfonates, alkyl sulfates and sulfonates, alkylbenzene sulfonates, alkyl and dialkyl sulfosuccinates, C₈-C₂₀ acyl isethionates, C₈-C₂₀ alkyl ether phosphates, C₈-C₂₀ sarcosinates, C₈-C₂₀ acyl lactylates, sulfoacetates and combinations thereof.

Useful amphoteric surfactants include cocoamidopropyl betaine, C₁₂-C₂₀ trialkyl betaines, sodium lauroamphoacetate and sodium laurodiamphoacetate.

Sunscreen actives may also be included in compositions of the present invention. These will be organic compounds having at least one chromophoric group absorbing within the ultraviolet ranging from 290 to 400 nm. Chromophoric organic sunscreen agents may be divided into the following categories (with specific examples) including: p-aminobenzoic acid, its salts and its derivatives (ethyl and isobutyl, glyceryl esters; p-dimethylaminobenzoic acid); anthranilates (o-aminobenzoates; methyl, menthyl, phenyl, benzyl, phenylethyl, linalyl, terpinyl and cyclohexenyl esters); salicylates (octyl, amyl, phenyl, benzyl, menthyl, glyceryl and dipropyleneglycol esters); cinnamic acid derivatives (menthyl and benzyl esters and alpha-phenyl cinnamonitrile; butyl cinnamoyl pyruvate); dihydroxycinnamic acid derivatives (umbelliferone, methylumbelliferone and methylaceto-umbelliferone); trihydroxycinnamic acid derivatives (esculetin, methylesculetin, daphnetin and the glucosides, esculin and daphnin); hydrocarbons (diphenylbutadiene and stilbene); dibenzalacetone and benzalacetophenone; naphtholsulfonates (sodium salts of 2-naphthol-3,6-disulfonic and of 2-naphthol-6,8-disulfonic acids); dihydroxy-naphthoic acid and its salts; o- and p-hydroxybiphenyldisulfonates; coumarin derivatives (7-hydroxy, 7-methyl and 3-phenyl); diazoles (2-acetyl-3-bromoindazole, phenyl benzoxazole, methyl naphthoxazole and various aryl benzothiazoles); quinine salts (bisulfate, sulfate, chloride, oleate and tannate); quinoline derivatives (8-hydroxyquinoline salts and 2-phenylquinoline); hydroxy- or methoxy-substituted benzophenones; uric and vilouric acids; tannic acid and its derivatives (e.g. hexaethylether); (butyl carbityl) (6-propyl piperonyl) ether; hydroquinone; and benzophenones (oxybenzone, sulisobenzone, dioxybenzone, benzoresorcinol, 2,2',4,4'-tetrahydroxybenzophenone, 2,2'-dihydroxy-4,4'-dimethoxybenzophenone, octabenzone, 4-isopropyldibenzoylmethane, butylmethoxydibenzoylmethane, etocrylene and 4-isopropyl-dibenzoylmethane). Particularly useful are: 2-ethylhexyl p-methoxycinnamate, 4,4'-t-butyl methoxydibenzoylmethane, 2-hydroxy-4-methoxybenzophenone, octyldimethyl p-aminobenzoic acid, digalloyltrioleate, 2,2-dihydroxy-4-methoxybenzophenone, ethyl 4-[bis(hydroxypropyl)]aminobenzoate , 2-ethylhexyl-2-cyano-3,3-diphenylacrylate, 2-ethylhexylsalicylate, glyceryl p-aminobenzoate, 3,3,5-trimethylcyclohexylsalicylate, methylanthranilate, p-dimethylaminobenzoic acid or aminobenzoate, 2-ethylhexyl p-dimethylaminobenzoate, 2-phenylbenzimidazole-5-sulfonic acid, 2-(p-dimethylaminophenyl)-5-sulfoniobenzoxazoic acid and mixtures thereof.

Particularly preferred are such materials as ethylhexyl p-methoxycinnamate, available as Parsol Mix®, Avobenzone, available as Parsol 1789®, and Dermablock OS® (octylsalicylate).

Amounts of the organic sunscreen agent will range from 0.1 to 15%, preferably from 0.5% to 10%, optimally from 1% to 8% by weight of the composition.

Advantageously present may also be water-insoluble organic material in the form of polymeric porous spherical particles. By the term "porous" is meant an open or closed cell structure. Preferably the particles are not hollow beads. Average particle size may range from 0.1 to 100, preferably from 1 to 50, more preferably greater than 5 and especially from 5 to 15 and optimally from 6 to about 10 µm. Organic polymers or copolymers are the preferred materials and can be formed from monomers including the acid, salt or ester forms of acrylic acid and methacrylic acid, methylacrylate, ethylacrylate, ethylene, propylene, vinylidene chloride, acrylonitrile, maleic acid, vinyl pyrrolidone, styrene, butadiene and mixtures thereof. The polymers are especially useful in cross-linked form. Cells of the porous articles may be filled by a gas which can be air, nitrogen or a hydrocarbon. Oil Absorbance (castor oil) is a measure of porosity and in the preferred but not limiting embodiment may range from 90 to 500, preferably from 100 to 200, optimally from 120 to 180 ml/100 grams. Density of the particles in the preferred but not limiting embodiment may range from 0.08 to 0.55, preferably from 0.15 to 0.48 g/cm³.

Illustrative porous polymers include polymethylmethacrylate and cross-linked polystyrene. Most preferred is polymethyl methacrylate available as Ganzpearl® GMP 820 available from Presperse Incorporated, Piscataway, New Jersey, known also by its INCI name of methyl methacrylate crosspolymer.

Amounts of the water-insoluble polymeric porous particles may range from 0.01 to 10%, preferably from 0.1 to 5%, optimally from 0.3 to 2% by weight of the composition.

Preservatives can desirably be incorporated into the compositions of this invention to protect against the growth of potentially harmful microorganisms. Particularly preferred preservatives are phenoxyethanol, methyl paraben, propyl paraben, imidazolidinyl urea, dimethyloldimethylhydantoin, ethylenediaminetetraacetic acid salts (EDTA), sodium dehydroacetate, methylchloroisothiazolinone, methylisothiazolinone, iodopropynbutylcarbamate and benzyl alcohol. The preservatives should be selected having regard for the use of the composition and possible incompatibilities between the preservatives and other ingredients. Preservatives are preferably employed in amounts ranging from 0.01% to 2% by weight of the composition.

A variety of herbal extracts may optionally be included in compositions of this invention. The extracts may either be water soluble or water-insoluble carried in a solvent which respectively is hydrophilic or hydrophobic. Water and ethanol are the preferred extract solvents. Illustrative extracts include those from green tea, chamomile, licorice, aloe vera, grape seed, citrus unshui, willow bark, sage, thyme and rosemary.

Also included may be such materials as lipoic acid, retinoxytrimethylsilane (available from Clariant Corporation under the Silcare 1M-75 trademark), dehydroepiandrosterone (DHEA), 1-piperidine propionic acid and combinations thereof. Ceramides (including Ceramide 1, Ceramide 3, Ceramide 3B and Ceramide 6) as well as pseudoceramides may also be useful. Amounts of these materials may range from 0.000001 to 10%, preferably from 0.0001 to 1% by weight of the composition.

Colorants, opacifiers and abrasives may also be included in compositions of the present invention. Each of these substances may range from 0.05 to 5%, preferably between 0.1 and 3% by weight of the composition.

Still other suitable actives for skin compositions and use in the present invention include creatine, resveratrol, hyaluronic acid (particularly those of molecular weight of around 800), and combinations thereof. Amounts may range from 0.000001 to 5%, preferably from 0.001 to 1% by weight of the compositions.

Compositions of the present invention may also contain vitamins. Illustrative watersoluble vitamins are niacinamide, vitamin B₂, vitamin B₆, vitamin C and biotin. Among the useful water-insoluble vitamins are vitamin A (retinol), vitamin A palmitate, ascorbyl tetraisopalmitate, vitamin E (tocopherol), vitamin E acetate and DL-panthenol. Total amount of vitamins when present in compositions according to the present invention may range from 0.001 to 10%, preferably from 0.01% to 1%, optimally from 0.1 to 0.5% by weight of the composition.

Desquamation agents are further optional components. Illustrative are the alpha-hydroxycarboxylic acids and beta-hydroxycarboxylic acids and salts of these acids. Among the former are salts of glycolic acid, lactic acid and malic acid. Salicylic acid is representative of the beta-hydroxycarboxylic acids. Amounts of these materials when present may range from 0.1 to 15% by weight of the composition.

Except in the operating and comparative examples, or where otherwise explicitly indicated, all numbers in this description indicating amounts of material ought to be understood as modified by the word "about".

The term "comprising" is meant not to be limiting to any subsequently stated elements but rather to encompass non-specified elements of major or minor functional importance. In other words the listed steps, elements or options need not be exhaustive. Whenever the words "including" or "having" are used, these terms are meant to be equivalent to "comprising" as defined above.

All documents referred to herein, including all patents, patent applications, and printed publications, are hereby incorporated by reference in their entirety in this disclosure.

The following examples will more fully illustrate the embodiments of this invention. All parts, percentages and proportions referred to herein and in the appended claims are by weight unless otherwise illustrated.

### EXAMPLE 1

A series of experiments were conducted to demonstrate the soft focus effects of formulations according to the present invention. Table I outlines the compositions of the evaluated formulations.

**TABLE I**

| Component | Sample Number (weight %) | | | | |
|---|---|---|---|---|---|
| | 1 | 2 | 3 | 4 | 5 |
| Viscoloam AT-100P® | 2.50 | 2.50 | 2.50 | 2.50 | 2.50 |
| Glydant Plus® Liquid | 0.20 | 0.20 | 0.20 | 0.20 | 0.20 |
| Gold | 0.02 | 0.02 | 0.02 | 0.02 | 0.02 |
| Acetamide MEA | -- | 0.50 | 1.00 | 2.00 | 4.00 |
| Water | qs | Qs | qs | qs | qs |

The base formulation included Viscoloam AT-100P® as a typical thickener. This material is a crosslinked polyacrylate. Glydant Plus® liquid is a preservative for the base formulation. It is a Lonza Incorporated trademark for a mixture of dimethyloldimethylhydantoin and iodopropynl butylcarbamate. Gold dust, obtained from Eytzinger Company, of average particle size 425 to 600 micron was utilized in all the experiments. Acetamide MEA is an acetamide monoethanolamine of structure (I) where R is methyl, sourced from Croda Incorporated as Incromectant® 100.

### Soft Focus Test Procedure

A 75 µm film of the formulation was applied to a glass slide using a Sheen cube film applicator. The film was dried for one day at room temperature. The coated slide was then placed in a gonioradiometer. The laser light source (wavelength 632 nm) was positioned at an angle of 48 degrees with the glass slide. The light source was turned on and measurement of the transmitted light intensity was made. Further measurements were made by sweeping the detector with an angular resolution of 3 degrees. The soft focus is calculated as the ratio of integration of transmittance from 9 to 90 degrees off the transmission angle and the integration from 0 to 90 degrees.

### Results

Samples 1-5 were evaluated via the soft focus procedure outlined above. Results are reported in table II.

**TABLE II**

| Sample No. | Acetamide MEA (weight %) | Soft Focus |
|---|---|---|
| 1 | 0.00 | 0.017 |
| 2 | 0.50 | 0.021 |
| 3 | 1.00 | 0.035 |
| 4 | 2.00 | 0.082 |
| 5 | 4.00 | 0.025 |

Soft focus values reported in Table II are the proportions of diffuse transmitted light (between 9 to 90 degree off incident angle). Higher values indicate better performance. Sample 1 is a control absent any acetamide MEA. Small amounts of acetamide MEA as in sample 2 (0.50%) provides a small soft focus boost. Increase to 1.00% of the alkanolamide reveals almost a doubling of the soft focus benefit as seen with the results on sample 3. A more than further doubling of benefit occurs at the 2.00% alkanolamide level shown in sample 4. The effect attenuates at much higher levels of additive. Sample 5 with 4.00% results in a benefit lying between those imparted by samples 2 and 3.

### EXAMPLES 2-6

Formulations suitable for the present invention are recorded in table III.

**TABLE III**

| Component | Example (weight %) | | | | |
|---|---|---|---|---|---|
| | 2 | 3 | 4 | 5 | 6 |
| Cyclopentasiloxane | 8.000 | 12.000 | 12.000 | 8.000 | 5.000 |
| Glycerin | 5.000 | 3.000 | 3.000 | 2.000 | 5.000 |
| Niacinamide | 5.000 | 5.000 | 5.000 | 5.000 | 5.000 |
| Ethyl hexyl methoxycinnamate | 5.000 | 5.000 | 5.000 | 5.000 | 5.000 |
| Cetearyl alcohol and ceteareth-20 | 2.200 | 2.200 | 2.200 | 2.200 | 2.200 |
| TiO2 and alumina and methicone | 2.000 | 2.000 | 2.000 | 2.000 | 2.000 |
| Butyl methoxydibenzoyl methane | 1.950 | 1.950 | 1.950 | 1.950 | 1.950 |
| PEG-100 stearate | 1.700 | 1.700 | 1.700 | 1.700 | 1.700 |
| Visolam AT 100P® | 1.500 | 1.500 | 1.500 | 1.500 | 1.500 |
| Dimethicone | 1.500 | 1.500 | 1.500 | 1.500 | 1.500 |
| Cholesterol | 1.000 | 1.000 | 1.4.00 | 1.400 | 1.400 |
| Behenyl alcohol | 0.500 | 0.500 | 0.500 | 0.500 | 0.500 |
| Isohexadecane | 0.500 | 0.500 | 0.500 | 0.500 | 0.500 |
| Stearic acid | 0.460 | 0.460 | 0.460 | 0.460 | 0.460 |
| Aristoflex HMB® | 0.350 | 0.350 | 0.350 | 0.350 | 0.350 |
| Caprylic/capric triglycerides | 0.250 | 0.250 | 0.250 | 0.250 | 0.250 |
| Butylated hydroxy toluene | 0.200 | 0.200 | 0.200 | 0.200 | 0.200 |
| Methyl paraben | 0.200 | 0.200 | 0.200 | 0.200 | 0.200 |
| Propyl paraben | 0.100 | 0.100 | 0.100 | 0.100 | 0.100 |
| Acetamide MEA | 0.050 | 0. 500 | 2.000 | 0. 500 | 1.000 |
| Gold 985 dust | 0.0002 | 0.002 | 0.020 | 0.0001 | 0.0005 |
| Water | Balance | Balance | Balance | Balance | Balance |

| | | | | | |
|---|---|---|---|---|---|
| (example 2 is not according to the invention) | | | | | |

## Claims

1. A cosmetic composition comprising:
i) from 0.00001 to 0.05% by weight of gold particles;
ii) from 0.5 to 4% by weight of an alkanolamide having structure I 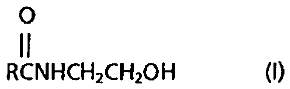 wherein R is a C₁-C₃ radical selected from the group consisting of methyl, ethyl, propyl, and isopropyl; and
iii) a cosmetically acceptable carrier which is water in an amount from 5 to 95% by weight of the composition.

2. A composition according to claim 1 wherein R is methyl.

3. A composition according to claim 1 or claim 2 wherein the gold particles have an average size ranging from 1 to 1,000 microns.

4. A composition according to any one of the preceding claims wherein the amount of the gold particles ranges from 0.0002 to 0.02% by weight.

5. A composition according to any one of the preceding claims wherein the gold particles are particles of a material selected from the group consisting of elemental gold, gold alloy and gold compounds.

6. A composition according to claim 5 wherein the material is gold alloy.

7. A composition according to claim 6 wherein the gold alloy is 22 karat gold.

8. A composition according to any one of the preceding claims wherein the gold particles are not supported on carrier particles nor are the gold particles coatings on carrier particles.

## Patentansprüche

1. Kosmetische Zusammensetzung, umfassend:
i) 0,00001 bis 0,05 Gew.-% Goldpartikel;
ii) 0,5 bis 4 Gew.-% eines Alkanolamids mit Struktur I 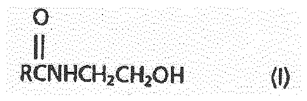 worin R ein C₁-C₃-Rest ist, der aus der Gruppe, bestehend aus Methyl, Ethyl, Propyl und Isopropyl, ausgewählt ist, und
iii) einen kosmetisch verträglichen Träger, der Wasser ist, in einer Menge von 5 bis 95 Gew.-% der Zusammensetzung.

2. Zusammensetzung gemäß Anspruch 1, wobei R Methyl ist.

3. Zusammensetzung gemäß Anspruch 1 oder Anspruch 2, wobei die Goldpartikel eine durchschnittliche Größe haben, die im Bereich von 1 bis 1000 Mikrometer liegt.

4. Zusammensetzung gemäß einem der vorangehenden Ansprüche, wobei die Menge der Goldpartikel im Bereich von 0,0002 bis 0,02 Gew.-% liegt.

5. Zusammensetzung gemäß einem der vorangehenden Ansprüche, wobei die Goldpartikel Partikel aus einem Material, ausgewählt aus der Gruppe, bestehend aus elementarem Gold, Goldlegierung und Goldverbindungen, sind.

6. Zusammensetzung gemäß Anspruch 5, wobei das Material Goldlegierung ist.

7. Zusammensetzung gemäß Anspruch 6, wobei die Goldlegierung 22-Karat-Gold ist.

8. Zusammensetzung gemäß einem der vorangehenden Ansprüche, wobei die Goldpartikel weder auf Trägerpartikel getragen sind noch Goldpartikelbeschichtungen auf Trägerpartikeln sind.

## Revendications

1. Composition cosmétique comprenant :
i) de 0,00001 à 0,05 % en poids de particules d'or ;
ii) de 0,5 à 4 % en poids d'un alcanolamide ayant la structure I 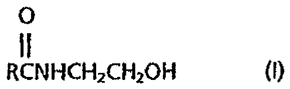 dans laquelle R est un radical en C₁ à C₃ choisi dans le groupe constitué du méthyle, de l'éthyle, du propyle et de l' isopropyle ; et
iii) un porteur acceptable sur le plan cosmétique qui est de l'eau dans une quantité de 5 à 95 % en poids de la composition.

2. Composition selon la revendication 1, dans laquelle R est le méthyle.

3. Composition selon la revendication 1 ou la revendication 2, dans laquelle les particules d'or ont une taille moyenne allant de 1 à 1 000 microns.

4. Composition selon l'une quelconque des revendications précédentes, dans laquelle la quantité des particules d'or va de 0,0002 à 0,02 % en poids.

5. Composition selon l'une quelconque des revendications précédentes, dans laquelle les particules d'or sont des particules d'un matériau choisi dans le groupe constitué de l'or élémentaire, d'un alliage d'or et de composés d'or.

6. Composition selon la revendication 5, dans laquelle le matériau est un alliage d'or.

7. Composition selon la revendication 6, dans laquelle l'alliage d'or est de l'or 22 carats.

8. Composition selon l'une quelconque des revendications précédentes, dans laquelle les particules d'or ne sont pas supportées sur des particules de porteur et les revêtements de particules d'or ne sont pas non plus supportés sur des particules de porteur.
